Europäisches Patentamt

European Patent Office

Office européen des brevets

(19)

(11) Veröffentlichungsnummer: **0 233 226**
**B1**

(12) **EUROPÄISCHE PATENTSCHRIFT**

(45) Veröffentlichungstag der Patentschrift: 25.07.90

(51) Int. Cl.⁵: **A 61 L 2/26**

(21) Anmeldenummer: **86904780.3**

(22) Anmeldetag: **08.08.86**

(86) Internationale Anmeldenummer:
**PCT/DE86/00324**

(87) Internationale Veröffentlichungsnummer:
**WO 87/01039 26.02.87 Gazette 87/05**

(54) VERFAHREN ZUM TESTEN VON DAMPFSTERILISATOREN SOWIE VORRICHTUNG ZUR DURCHFÜHRUNG DES VERFAHRENS.

(30) Priorität: **12.08.85 DE 8523448 u**

(43) Veröffentlichungstag der Anmeldung:
**26.08.87 Patentblatt 87/35**

(45) Bekanntmachung des Hinweises auf die Patenterteilung:
**25.07.90 Patentblatt 90/30**

(84) Benannte Vertragsstaaten:
**DE**

(56) Entgegenhaltungen:
**US-A-4 486 387**

(73) Patentinhaber: **TOLZIN, Hedwig**
**Poststrasse 83**
**D-4048 Grevenbroich 2 (DE)**

(72) Erfinder: **TOLZIN, Hedwig**
**Poststrasse 83**
**D-4048 Grevenbroich 2 (DE)**

(74) Vertreter: **Bonsmann, Manfred, Dipl.-Ing.**
**Kaldenkirchener Strasse 35a**
**D-4050 Mönchengladbach 1 (DE)**

Courier Press, Leamington Spa, England.

**Beschreibung**

Die Erfindung betrifft ein Verfahren zum Testen von Dampfsterilisatoren, bei dem in den Sterilisator ein Testmaterialstapel mit einem in dessen Innerem eingelegten, chemische Indikatoren tragenden Testbogen eingebracht wird, und dann ein bestimmter Sterilisationsprozeß abläuft, und dann der Testmaterialstapel entnommen und der Testbogen aus dem Testmaterialstapel entnommen und das Testergebnis anhand des Zustandes der chemischen Indikatoren (z.B. Farbumschlag) festgestellt wird.

Es ist bekannt, für die Überwachung der Dampfsterilisation zum Sterilisieren im medizinischen Bereich chemische Indikatoren einzusetzen, welche im Inneren eines Testmaterialstapels angeordnet sind. Üblicherweise wird ein chemische Indikatoren tragender Testbogen in die Mitte eines Testpaketes eingebracht, welches aus einer vorherbestimmten Anzahl von Lagen gefalteter Wäsche besteht. Es ist auch vorgeschlagen worden, einen nicht aus Textilien, wie beispielsweise Leinentüchern, sondern aus gestapelter Pappe bestehenden Testmaterialstapel einzusetzen, wobei die Pappe eine bestimmte Porosität aufweist.

Die bei Dampfsterilisatoren am häufigsten auftretende Störung ist eine "schleichende Verschlechterung des Vakuums", die darauf beruht, daß die Vakuumeinrichtung eines Sterilisators (Vakuumpumpe und Dichtungen) einem Verschleiß unterliegt. Um festzustellen, ob ein Sterilisator noch den an ihn gestellten Anforderungen genügt, wird ein Testmaterialstapel der beschriebenen Art einem Sterilisiervorgang unterworfen. Nach Abschluß des Sterilisiervorganges können dann die interessierenden Betriebseigenschaften des Sterilisators durch Betrachten des während des Tests in dem Testmaterialstapel befindlichen Testbogens festgestellt werden.

Aus der US-A-4 486 387 ist es bekannt, einen aus Pappe bestehenden, den Testbogen enthaltenden Testmaterialstapel vor dem Sterilisationsvorgang paketartig in ein Sterilisationspapier einzuschlagen.

Es wurde festgestellt, daß Tests bei gleichem Zustand des jeweiligen Sterilisators zu unterschiedlichen Ergebnissen führen können.

Der Erfindung liegt die Aufgabe zugrunde, hier Abhilfe zu schaffen und Maßnahmen vorzuschlagen, mittels derer auf einfache Weise erreicht werden kann, daß die Teste zu vergleichbaren Ergebnissen führen.

Zur Lösung dieser Aufgabe wird vorgeschlagen, daß ein lose aufeinanderliegende Lagen aufweisender Testmaterialstapel eingebracht wird, und daß der Stapel während des Sterilisationsprozesses mittels auf die Stapelhöhe einwirkender mechanischer Druckhaltemittel unter flächenmäßig im wesentlichen gleichen Druckbedingungen gehalten wird, so daß sich während des Sterilisationsprozesses keine Aufwölbungen, Kanäle od. dgl. in dem Testmaterialstapel bilden.

Dadurch wird erreicht, daß die Druckbedingungen in einem Testmaterialstapel zwischen einzelnen Tests immer gleich gehalten werden können. Die sich bei dem Sterilisierprozeß zwischen der sog. Vorvakuumphase und der eigentlichen Sterilisierphase unkontrolliert bildenden Aufwölbungen, Kanäle od. dgl. in dem Testmaterialstapel, die das Testergebnis verfälschen können, werden auf diese Weise vermieden, da entsprechend dem erfindungsgemäßen Vorschlag das Testpaket in dem Sterilisator stets gleichen physikalischen Druckbedingungen unterliegt.

Ein weiterer Vorteil besteht in folgendem: Bisher gebräuchliche Testmaterialstapel aus Pappe werden paketartig in ein Papier eingeschlagen geliefert und können nur einmal verwendet werden. Dies liegt daran, daß die Pappe durch die in Dampfsterilisatoren herrschende Feuchtigkeit aufweicht und sich aufwölbt, so daß nach erstmaligem Gebrauch Aufwölbungen, Kanäle od. dgl. in dem Stapel gebildet sind, die bei einem nochmaligen Gebrauch des Testmaterialstapels (natürlich mit neuem Testbogen) zu einem gegenüber dem ersten Testergebnis unterschiedlichen Resultat führen. Da erfindungsgemäß die Höhe des Testmaterialstapels während des Sterilisationsvorganges zwangsweise begrenzt wird, so daß sich keine Aufwölbungen und damit Luftinseln bilden können und das Bild, was sich nach dem Test auf dem Testbogen zeigt, stets ein vergleichbares Bild zu dem vorherigen Testergebnis bei Überprüfung des gleichen Sterilisators ergibt, kann der gleiche Stapel (natürlich mit jeweils neuem Testbogen) mehrfach verwendet werden. Man kann nämlich erst unter Zugrundelegung gleicher Testbedingungen eine zuverlässige Aussage darüber machen, ob der Sterilisator in einem einwandfreien Zustand ist bzw. nicht ist.

Der nach dem Stand der Technik bekannte Test von Dampfsterilisatoren, auch "Bowie-Dick-Test" genannt, wird üblicherweise nur in zeitlichen Abständen durchgeführt, beispielsweise einmal pro Tag. In vielen Fällen erfolgt in der Zwischenzeit keinerlei Kontrolle des Sterilisators. Bei manchen Sterilisatoren wird während des laufenden Sterilisationsbetriebes ein Druck- und Temperatur-Diagramm aufgenommen. Derartige Diagramme bieten aber keine hundertprozentige Garantie dafür, daß der Sterilisationsprozeß stets ordnungsgemäß verläuft. Weiterhin ist es auch bekannt, in dem normalen Sterilisiergut Indikatoren unterzubringen. Der Zustand dieser Indikatoren wird jedoch oft erst tagelang nach dem Sterilisationsvorgang geprüft und gibt somit für den laufenden Betrieb keinen Aufschluß über den Zustand des Sterilisators.

In weiterer Ausgestaltung der Erfindung wird demgegenüber vorgeschlagen, daß der erfindungsgemäß vorgesehene Test während bzw. auch während des laufenden Sterilisationsbetriebes durchgeführt wird. Dabei sollte dem Umstand Rechnung getragen werden, daß die Ansprechempfindlichkeit des Teststapels für den Test bei laufendem Sterilisationsbetrieb und bei ansonstem leerem Sterilisator unterschiedlich gewählt wird.

Für die Durchführung des Verfahrens findet zweckmäßigerweise eine Vorrichtung Verwendung, mittels derer die Begrenzung der Höhe eines lose aufeinanderliegende Lagen aufweisenden Testmmaterialstapels erreicht werden kann. Diese Vorrichtung ist als Aufnahmeeinrichtung ausgebildet und weist je ein mit Durchbrechungen versehenes und über einen Aufnahmeraum für den Testmaterialstapel umschließende und zur Begrenzung der Höhe des Testmaterialstapels ausgebildete Abstandseinrichtungen verbindbares Bodenteil und Deckelteil aufweist. Die in dem Bodenteil und Deckelteil vorgesehenen Durchbrechungen dienen dazu, einen Dampfeintritt in den Testmaterialstapel zu ermöglichen. Der Auflagedruck auf den Testmaterialstapel, welcher üblicherweise in horizontaler Lage zum Testen in den Sterilisator eingebracht wird, soll möglichst gleichmäßig sein. Dies kann dadurch erreicht werden, daß das Bodenteil bzw. Deckelteil als Lochblech oder auch als siebartiges Drahtgeflecht ausgebildet ist. Vorzugsweise können oberhalb bzw. unterhalb des die Durchbrechungen aufweisenden Flächengebildes des Bodenteils oder Deckelteils Verstrebungen od. dgl. angeordnet sein.

In vorteilhafter Ausgestaltung der Erfindung kann vorgesehen sein, daß die Abstandseinrichtung an der Innenseite des Deckelteils und/oder Bodenteils angebrachte und ineinander bzw. in Aufnahmeöffnungen einrastbare Verschlußzapfen aufweisen.

Weiterhin kann erfindungsgemäß vorgesehen sein, daß die Abstandseinrichtungen in ihrer Höhe und/oder Klemmkraft einstellbar ausgebildet sind.

Bei Einsatz der erfindungsgemäßen Vorrichtung kann somit erreicht werden, daß der Testmaterialstapel während des Testvorganges definiert eingeklemmt bzw. in seiner Höhe begrenzt wird.

Ein Testmaterialstapel zur Verwendung bei dem Verfahren gemäß der Erfindung bzw. zum Einsatz in eine Vorrichtung zur Durchführung des Verfahrens ist dadurch gekennzeichnet, daß ein loser Stapel von nicht gewebten porösen Flächengebilden vorgesehen ist, und die zur Aufnahme des Testbogens vorgesehene Mitte dieses Stapels durch eine Markierung gekennzeichnet ist.

Die Markierung kann beispielsweise durch ein seitlich vorstehendes Blatt erfolgen, welches vor der Verwendung des Stapel herausgenommen und durch den Testbogen ersetzt wird. Es können aber auch beispielsweise farbige Markierungen vorgesehen sein, so etwa farblich unterschiedlich ausgebildete Stapelhälften.

Als besonders zweckmäßig hat sich gezeigt, aus Papier bzw. auf Papierbasis hergestellte Flächengebilde zu verwenden. Der kritische Zustand des Stapels hängt insbesondere von dem Retentionswert der porösen Flächengebilde ab. Erfindungsgemäß wird vorgeschlagen, daß der Retentionswert der verwendeten Flächengebilde in der Größenordnung von 99% nach Britisch Standard BS 4400 liegt.

Wie bereits erwähnt, besteht ein besonderer Vorteil der Erfindung darin, daß ein Mehrfachgebrauch des Testmaterialstapels (natürlich stets mit neuem Testbogen) möglich ist.

In den Zeichnungen ist ein Ausführungsbeispiel der erfindungsgemäßen Vorrichtung (Aufnahmeeinrichtung) schematisch dargestellt. Es zeigen:

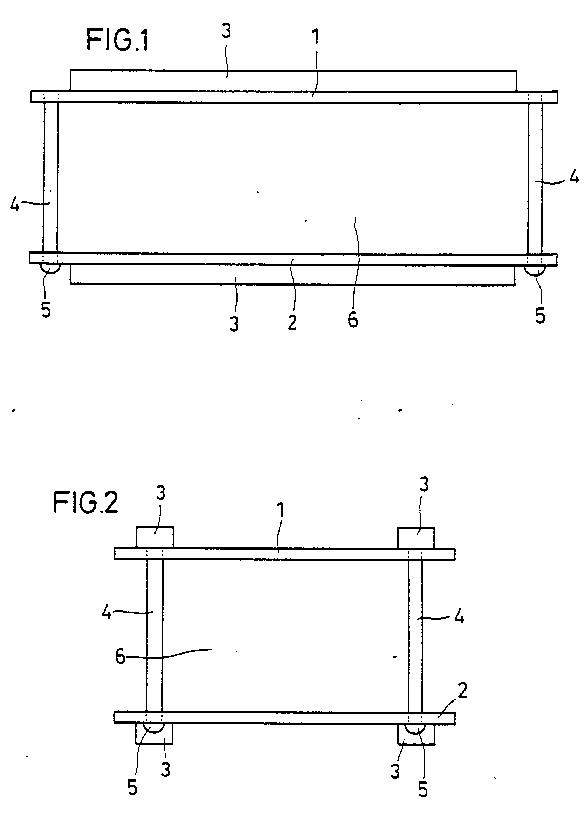

Fig. 1 eine schematische Seitenansicht der Vorrichtung im Arbeitszustand;

Fig. 2 eine schematische Ansicht der Stirnseite der Vorrichtung gemäß Fig. 1;

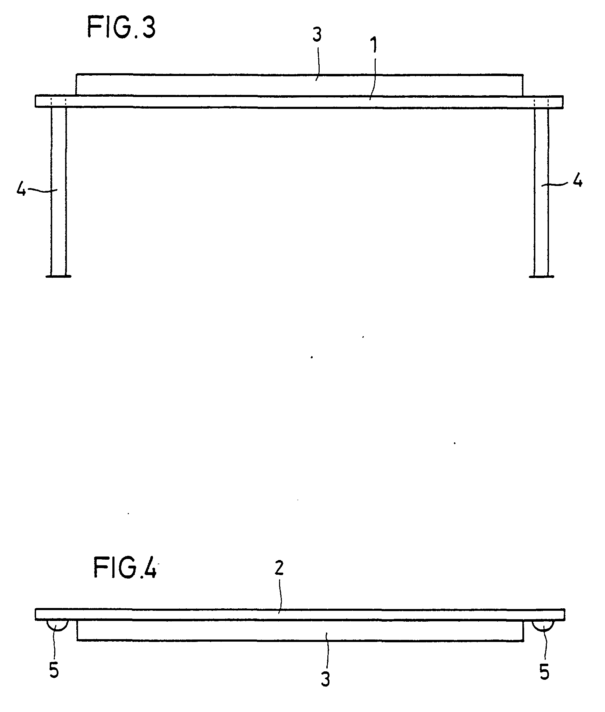

Fig. 3 eine schematische Seitenansicht der Längsseite des Deckelteils der Vorrichtung gemäß Fig. 1 mit daran angebrachten Verschlußzapfen;

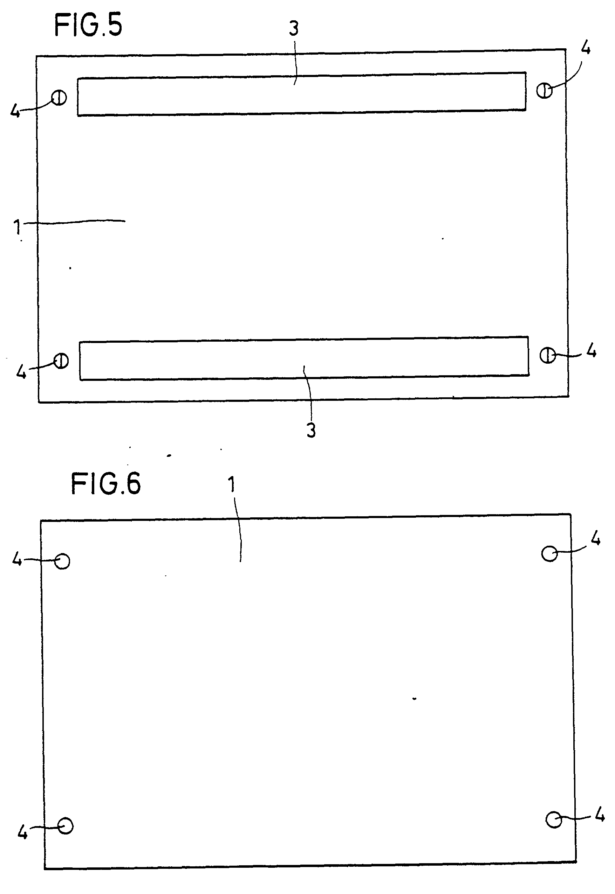

Fig. 4 eine schematische Seitenansicht der Längsseite des Unterteils der Vorrichtung gemäß Fig. 1;

Fig. 5 eine schematische Draufsicht auf die Oberseite des Deckelteils;

Fig. 6 eine schematische Draufsicht auf die Unterseite des Deckelteils;

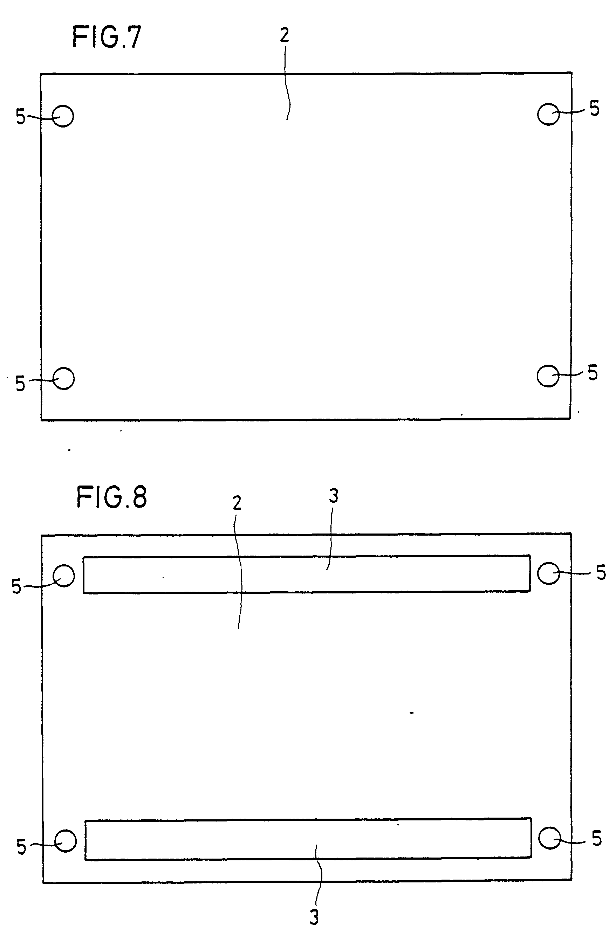

Fig. 7 eine schematische Draufsicht auf die Oberseite des Bodenteils;

Fig. 8 eine schematische Draufsicht auf die Unterseite des Bodenteils.

Ein mit 1 bezeichnetes Oberteil und ein mit 2 bezeichnetes Unterteil ist jeweils als Lochblech ausgebildet.

Auf der Oberseite des Oberteils 1 und auf der Unterseite des Unterteils 2 sind U-förmig ausgebildete Verstärkungsprofile angebracht. Die an der Unterseite des Bodenteils angeordneten Verstärkungsprofile 3 dienen gleichzeitig als Fuß der Vorrichtung. An der Unterseite des Deckelteils 3 sind jeweils in den Eckbereichen stabartige bzw. säulenartige Verschlußzapfen angeordnet, welche sich quer zu der Ebene des Deckelteils auf der den Verstärkungsprofilen gegenüberliegenden Seite erstrecken und welche in Haltenocken 5 einrasten können, die an entsprechenden Stellen des Bodenteils 2 angeordnet sind. In eingerastetem Zustand wird von dem Bodenteil 2 und dem Deckelteil 1 sowie den in den Eckbereichen vorgesehenen Abstandseinrichtungen ein Raum 6 umschlossen, der zur Aufnahme eines (nicht dargestellten) Testmaterialstapels zum Testen von Dampfsterilisatoren besteht. Der Testmaterialstapel besteht aus einer Vielzahl von Lagen aus Wäsche oder Pappe und wird durch die erfindungsgemäße Einrichtung stets unter einem definierten bzw. definierbaren Druck gehalten.

Bei dem beschriebenen Ausführungsbeispiel wird die Begrenzung der Höhe des Testmaterialstapels bzw. die Aufrechterhaltung eines definierten Druckes durch mechanische Einrichtungen bewirkt, die im wesentlichen aus den in die Haltenocken 5 einrastbaren Verschlußzapfen 4 bestehen.

Es liegt aber auch im Rahmen der Erfindung, wenn andere mechanisch ausgebildete Klemmeinrichtungen, wie beispielsweise Schnappverbindungen od. dgl. eingesetzt werden, auch solche, welche in ihrer Höhe und/oder Klemmkraft einstellbar ausgebildet sind. Es können auch anderweitig ausgebildete Druckerzeugungs- bzw. Höhenbegrenzungseinrichtungen im Rahmen der Erfindung Verwendung finden.

Ein weiterer Vorteil der Erfindung besteht in folgendem: Wenn das Vorhandensein von Luft im Testmaterial angezeigt wird, so können die Ursachen hierfür unterschiedlich sein. Ein Grund kann darin bestehen, daß eine unzureichende Entlüftung erfolgte, d.h., daß das tiefe Vakuum nicht erreicht wurde. Ein weiterer Grund kann darin bestehen, daß die Sterilisierkammer undicht ist. Schließlich kann der Grund auch darin liegen, daß sich Luft im Dampf befunden hat.

Durch die Erfindung ist nun die Möglichkeit gegeben, in einfacher Weise durch eine "Gegenprobe" weitgehend die Ursache des Fehlers zu ermitteln. Wird nämlich Luft festgestellt, so kann anschließend ein weiterer Test mit verkleinertem Testmaterial (sog. "Gegenprobe") durchgeführt werden. Wird bei dieser Gegenprobe keine Luftinsel mehr festgestellt, so bestand die Ursache in einer unzureichenden Entlüftung, d.h., das tiefe Vakuum wurde nicht erreicht, und es kann eine entsprechende Reparatur erfolgen.

**Patentansprüche**

1. Verfahren zum Testen von Dampfsterilisatoren, bei dem in den Sterilisator ein Testmaterialstapel mit einem in dessen Inneren eingelegten, chemische Indikatoren tragenden Testbogen eingebracht wird und dann ein bestimmter Sterilisationsprozeß abläuft, und dann der Testmaterialstapel entnommen und der Testbogen aus dem Testmaterialstapel entnommen und das Testergebnis anhand des Zustandes der chemischen Indikatoren (z. B. Farbumschlag) festgestellt wird, dadurch gekennzeichnet, daß ein lose aufeinanderliegende Lagen aufweisender Testmaterialstapel eingebracht wird, und daß der Stapel während des Sterilisationsprozesses mittels auf die Stapelhöhe einwirkender mechanischer Druckhaltemittel unter flächenmäßig im wesentlichen gleichen Druckbedingungen gehalten wird, so daß sich während des Sterilisationsprozesses keine Aufwölbungen, Kanäle od. dgl. in dem Stapel bilden.

2. Verfahren nach Anspruch 1, dadurch gekennzeichnet, daß der Test während des laufenden Sterilisationsbetriebes durchgeführt wird.

3. Vorrichtung für die Begrenzung der Höhe eines lose aufeinanderliegende Lagen aufweisenden Testmaterialstapels zur Verwendung gemäß Anspruch 1 oder 2, dadurch gekennzeichnet, daß die Aufnahmeeinrichtung je ein mit Durchbrechungen versehenes und über einen Aufnahmeraum (6) für den Testmaterialstapel umschließende und zur Begrenzung der Höhe des Testmaterialstapels ausgebildete Abstandseinrichtungen (4, 5) verbindbares Bodenteil (2) und Deckelteil (1) aufweist.

4. Aufnahmeeinrichtung nach Anspruch 3, dadurch gekennzeichnet, daß das Bodenteil (2) und/oder das Deckelteil (1) als Lochblech ausgebildet ist bzw. sind.

5. Aufnahmeeinrichtung nach Anspruch 3, dadurch gekennzeichnet, daß das Bodenteil (2) und/oder das Deckelteil (1) ein siebartiges Drahtgeflecht aufweist bzw. aufweisen.

6. Aufnahmeeinrichtung nach einem der Ansprüche 3 bis 5, dadurch gekennzeichnet, daß die Abstandseinrichtungen an der Innenseite des Bodenteils (2) und/oder des Deckelteils (1) angebrachte, ineinander bzw. in Aufnahmeöffnungen (Haltenocken 5) einrastbare Verschlußzapfen (4) aufweisen.

7. Aufnahmeeinrichtung nach einem der Ansprüche 3 bis 6, dadurch gekennzeichnet, daß die Abstandseinrichtungen (4) in ihrer Höhe und/oder Klemmkraft einstellbar ausgebildet sind.

8. Testmaterialstapel zur Verwendung in dem Verfahren gemäß Anspruch 1 oder 2 bzw. in einer Vorrichtung gemäß einem oder mehreren der Ansprüche 3 bis 7, dadurch gekennzeichnet, daß ein loser Stapel von nicht gewebten porösen Flächengebilden vorgesehen ist, und die zur Aufnahme des Testbogens vorgesehene Mitte dieses Stapels durch eine Markierung gekennzeichnet ist.

9. Testmaterialstapel nach Anspruch 8, dadurch gekennzeichnet, daß der Retentionswert der verwendeten Flächengebilde in der Größenordnung von 99% nach British Standard BS 4400 liegt.

**Revendications**

1. Méthode de test pour autoclaves de stérilisation, qui comprend le placement dans l'autoclave d'un paquet de test contenant à l'intérieur une feuille de test pourvue d'indicateurs chimiques, ensuite le déroulement d'un processus déterminé de stérilisation, ensuite la prise du paquet de test de l'autoclave et la prise de la feuille de test du paquet de test, et la détermination du résultat du test au moyen de la condition des indicateurs (changement de couleur, p.ex.); caractérisée par le fait qu'un paquet de test composé de couches superposées de façon lâche est placé dans l'autoclave, et que le paquet, pendant le processus de stérilisation, est soumis pour toute sa surface à des conditions de pression maintenues essentiellement stables par moyen d'un dispositif mécanique de maintien de pression agissant sur la pile de manière à supprimer la formation de bombements, de canaux, etc.

2. Méthode selon la revendication no. 1, caractérisée par le fait que le test est exécuté pendant la stérilisation en cours.

3. Dispositif de limitation de la hauteur d'un paquet de test composé de couches superposées de façon lâche, pour l'utilisation selon la revendication no. 1 ou no. 2, caractérisé par le fait que le dispositif de réception est constitué par une partie inférieure (2) et une partie supérieure (1) perforées qui peuvent être raccordées par des dispositifs d'écartement (4, 5) enfermant une espace de réception (6) du paquet de test et destinés à limiter la hauteur du paquet de test.

4. Dispositif de réception selon la revendication no. 3, caractérisé par le fait que la partie inférieure (2) et/ou la partie supérieure (1) est/sont fabriquée/s de tôle perforée.

5. Dispositif de réception selon la revendication no. 3, caractérisé par le fait que la partie inférieure (2) et/ou la partie supérieure (1) comprend/comprennent un tressage de fil métallique comme un crible.

6. Dispositif de réception selon une des revendications no. 3 à no. 5, caractérisé par le fait que les dispositifs d'écartement présentent des tenons de fermeture (4) montés à l'intérieur de la partie inférieure (2) et/ou de la partie supérieure (1), apts à encliqueter l'un dans l'autre ou dans des ouvertures de réception (cames de retenue (5)).

7. Dispositif de réception selon une des revendications no. 3 à no. 6, caractérisé par le fait que les dispositifs d'écartement (4) sont ajustables quant à leur hauteur et/ou force de serrage.

8. Paquet de test pour l'utilisation dans le procédé selon la revendication no. 1 ou no. 2 resp. dans un dispositif selon une ou plusieurs des revendications no. 3 à no. 7, caractérisé par le fait qu'une pile lâche de feuilles poreuses non-tissées est prévue, et que le milieu de cette pile destiné à la réception de la feuille de test présente une marque.

9. Paquet de test selon la revendication no. 8, caractérisé par le fait que la rétention des feuilles utilisées se chiffre à 99% selon le British Standard BS 4400.

## Claims

1. A steam sterilizer testing method involving the placement of a test pack which comprises inside a test sheet bearing chemical indicators into the sterilizer, thereupon a specific sterilization process, thereupon removal of the test pack from the sterilizer and removal of the test sheet from the test pack, and determination of the test result by the chemical indicators' condition (e.g. color changes); characterized in that a test pack consisting of loosely superimposed layers is placed into the sterilizer, and in that during the sterilization process the pack is kept under substantially constant compressive conditions on its whole surface by means of a mechanical pressure maintenance device acting on the stack, thus preventing the formation of bulges, ducts, etc. in the pack during the sterilization process.

2. A method according to Claim 1, characterized in that the test is conducted during the sterilization in process.

3. A device for limiting the height of a test pack consisting of loosely superimposed layers to be used according to Claim 1 or 2, wherein the receiving device consists of a perforated shell each for the bottom (2) and for the cover (1) which can be connected by separators (4, 5) enclosing a receiving space (6) for the test pack and designed for limiting the height of the test pack.

4. A receiving device according to Claim 3, wherein the bottom shell (2) and/or the cover shell (1) is/are made from perforated plate.

5. A receiving device according to Claim 3, wherein the bottom shell (2) and/or the cover shell (1) has/have a sieve-like wire network.

6. A receiving device according to one of the Claims 3 to 5, wherein the separators have locking plugs (4) fitted to the inside of the bottom shell (2) and/or the cover shell (1), which snap into each other resp. into inlets (holding cams (5)).

7. A receiving device according to one of the Claims 3 to 6, wherein the separators (4) are adjustable in their height and/or clamping power.

8. A test pack for use in the process according to Claim 1 or 2 resp. in a device according to one or more of the Claims 3 to 7, wherein a loose stack of nonwoven porous sheets is provided for, and the middle of this stack where the test sheet is intended to be placed shows a mark.

9. A test pack according to Claim 8, wherein the retention of the sheets used runs to 99% according to British Standard BS 4400.

FIG.1

FIG.2

FIG.3

FIG.4

FIG.5

FIG.6

FIG.7

2

5

5

5

5

FIG.8

2    3

5

5

3

5

5

3